# EUROPÄISCHE PATENTSCHRIFT

(11) **EP 2 689 749 B1**
(45) Veröffentlichungstag und Bekanntmachung des Hinweises auf die Patenterteilung: **08.06.2016**
(21) Anmeldenummer: 12178008.4
(22) Anmeldetag: 26.07.2012
(51) Int. Cl.: A61F 2/30, A61F 2/36, A61F 2/38, A61B 17/72

(54) **Aufsteckmodul für eine Langschaftprothese**
Clip-on module for a long shaft prosthetic
Module de montage pour une prothèse à longue tige

(43) Veröffentlichungstag der Anmeldung: 29.01.2014
(73) Patentinhaber: WALDEMAR LINK GmbH & Co. KG, 22339 Hamburg (DE)
(72) Erfinder: Link, Helmut D., 22397 Hamburg (DE)
(74) Vertreter: Glawe, Delfs, Moll

(56) Entgegenhaltungen:
- EP-A2- 1 358 860
- WO-A2-2008/048195
- FR-A1- 2 788 429
- US-A1- 2004 254 646

## Beschreibung

Die Erfindung betrifft ein System aus einem zur Implantation in einem Lang gestreckten Knochen ansgebildeten schaft und einem Aufsteckmodul zum Aufschieben auf ein freies Ende des Schafts, umfassend einen Haltekörper mit einer Axialbohrung für den Schaft an dem unteren Ende des Haltekörpers und einen Gelenkkopf als ein Teil einer Gelenkendoprothese.

Ein solches System ist aus WO 2008/048195 A2 bekannt.

Erkrankungen im Bereich der großen Knochen, insbesondere in den Extremitäten, bedingen häufig die Implantation einer langschaftigen Endoprothese, die zur Verstärkung des Knochens dient und die in vielen Fällen gleichzeitig auch als Verankerung für ein Gelenkelement einer Gelenkendoprothese dient, beispielsweise einer Knieprothese. Im Regelfall wird der Schaft von der Seite in den Knochen, beispielsweise einen Oberschenkelknochen (Femur), eingebracht, an der sich die pathologische Stelle befindet. Im Fall einer Kniegelenkendoprothese bedeutet dies, dass der Schaft üblicherweise von dem knieseitigen Ende des Oberschenkelknochens (distales Ende des Femur) eingebracht wird. Es gibt aber insbesondere bei speziellen Pathologien auch Fälle, in denen der Schaft von dem gegenüberliegenden Ende aus eingebracht wird (im Fall der Kniegelenkendoprothese also von dem proximalen Ende des Femur), und dann durch den Markkanal des Knochens durchgeschoben wird. Derartige Schäfte sind unter der Bezeichnung "Durchsteckschaft" allgemein bekannt.

Es kann nun der Fall auftreten, dass an dem anderen Ende des Durchsteckschafts ebenfalls eine Pathologie auftritt, die eine prothetische Versorgung erfordert. Am Beispiel der Kniegelenkendoprothese heißt dies, dass eine Pathologie im Bereich des Hüftgelenks auftritt und somit ggf. auch nachträglich die Implantation einer Hüftgelenkendoprothese indiziert ist. In der Praxis bedeutet dies häufig, dass der Durchsteckschaft ausgewechselt werden muss. Dies ist eine aufwändige Operation, bei der es häufig an dem ohnehin bereits durch die Pathologien geschwächten Knochen noch zu weiterem Substanzverlust kommt. Besonders schwierig sind jene Fälle, bei denen es bereits zu Brüchen im geschädigten Gelenksbereich gekommen ist (beispielsweise ein Schenkelhalsbruch hinsichtlich des Hüftgelenks), wodurch sich in der Folge auch unterschiedliche Längen der Extremitäten ergeben können. Dies ist für die Patienten ein schwerer Nachteil.

Der Erfindung liegt die Aufgabe zu Grunde, diese Nachteile zu verringern und eine Prothese der eingangs genannten Art universeller verwendbar zu machen.

Die erfindungsgemäße Lösung liegt in den Merkmalen der unabhängigen Ansprüche. Vorteilhafte Weiterbildungen sind Gegenstand der abhängigen Ansprüche.

Bei einem System gemäß den Merkmalen des Oberbegriffs des Anspruchs 1 ist erfindungsgemäß vorgesehen, dass das Kopfelement in Axialrichtung in der Weise soweit von dem oberen Ende des Haltekörpers nach unten versetzt ist, dass der Schaft über den Gelenkkopf nach oben hinausragt. Der Längenbetrag, um den der Schaft über den Gelenkkopf nach oben hinaus ragt, wird als Versatz bezeichnet.

Unter Gelenkkopf wird eine der Komponenten eines Prothesengelenks verstanden, welche die Position des Bewegungszentrums bestimmt. Bei einem Kugelgelenk (beispielsweise einer Hüftprothese) ist es das Zentrum der Gelenkskugel, wobei bei einer zweiteiligen Ausführung mit einem am Haltekörper angeordneten Grundkonus und einem aufsteckbaren Kopf der Grundkonus für die Position des Bewegungszentrums bestimmend ist. Bei einem Scharniergelenk (beispielsweise einer Knieprothese) ist es die Lage der Rotationsachse, die für die Position des Bewegungszentrums bestimmend ist.

Unter Axialrichtung wird die Richtung verstanden, in die der Schaft in die Axialbohrung einzusetzen ist. Die Angaben "unten" und "oben" beziehen sich auf diese Axialrichtung.

Kern der Erfindung ist der Gedanke, den Gelenkkopf nach unten, also zum Schaft hin, versetzt anzuordnen. Der Gelenkkopf befindet sich also nicht wie üblich oberhalb des Haltekörpers an dem freien Ende des Schaftes, sondern befindet sich vielmehr unterhalb in quasi versenkter Position. Dank dieser "versenkten" Anordnung vergrößert sich das Einsatzspektrum für das Aufsteckmodul auf solche Fälle, in denen bisher ein kürzerer Durchsteckschaft erforderlich gewesen wäre und deshalb eine aufwändige Revisionsoperation zum Austausch des Durchsteckschafts hätte durchgeführt werden müssen. Dies kann mit dem erfindungsgemäßen Aufsteckmodul dank der "versenkten" Anordnung des Gelenkkopfs vermieden werden. Der Patient wird damit beträchtlich geschont. Außerdem verringert sich die Gefahr von Komplikationen. Die Erfindung vereint damit Vorteile hinsichtlich geringeren Aufwands mit knochensubstanzsparender Operationstechnik und größerer Schonung des Patienten.

Vorzugsweise ist der Gelenkkopf zusätzlich in eine Richtung quer zur Axialrichtung versetzt. Hierbei kann es sich insbesondere um eine Verkippung nach anterior oder posterior handeln. Damit können auch solche Fälle mit der erfindungsgemäßen Prothese versorgt werden, bei denen es aufgrund der Pathologie zu einer Änderung des Anteversionswinkels gekommen ist.

Vorzugsweise ist die Axialbohrung zur Aufnahme des Schaftes als Sackloch ausgeführt. Damit wird auf einfache Weise eine exakte Positionierung des Aufsteckmoduls in Relation zu dem Schaft erreicht. Alternativ kann die Axialbohrung auch als gestufte Durchgangsbohrung ausgeführt sein, wobei eine Anlageschulter vorgesehen ist und jenseitig davon die Weite der Bohrung kleiner ist als die Weite des Schaftes. Der jenseitige Teil dient zur Aufnahme einer Sicherungsschraube, welche den Schaft an dem erfindungsgemäßen Aufsteckmodul fixiert.

Mit Vorteil ist am Boden des Sacklochs oder im Bereich der Anlageschulter eine Radialverzahnung ausgebildet. Sie ist zweckmäßigerweise als Vielfachverzahnung ausgeführt mit einer Winkelabstufung von vorzugsweise mindestens 1° und höchstens 5°. Damit kann das Aufsteckmodul präzise und hinsichtlich seiner Rotationsposition in Bezug auf den Schaft sicher positioniert und befestigt werden. Dank der versenkten Anordnung der Vielfachverzahnung besteht keine Gefahr einer Irritation von umliegendem Gewebe.

Vorzugsweise ist der Gelenkkopf zweiteilig ausgeführt, nämlich mit einem am Haltekörper angeordneten Grundkonus und einem aufsteckbaren Kopf. Dies ermöglicht zum einen eine einfache Montage und zum anderen eine Variation des Kopfdurchmessers bzw. der Lage des Kopfes durch Wahl von anderen Köpfen mit unterschiedlicher Einstecktiefe für den Grundkonus. Die Variabilität wird damit deutlich erhöht.

In den meisten Fällen wird der Gelenkkopf starr am Haltekörper ausgebildet sein. Es ist aber auch möglich, dass der Gelenkkopf positionsvariabel am Haltekörper angeordnet ist, um so unterschiedliche Versatzmaße einzustellen. Damit kann mit lediglich einer Prothese eine Vielzahl von Anwendungsfällen mit entsprechend unterschiedlichen Versatzmaßen abgedeckt werden.

Zweckmäßigerweise ist am Gelenkkopf ein in kaudal-medialer Richtung abragenden kragenartiger Vorsprung vorgesehen. Er fungiert als Auflage und kann somit die insbesondere vom Patienten beim Stehen oder bei Laufbewegung von der Prothese eingeleitete Aufstandskraft besser auf den Femur übertragen.

Zweckmäßigerweise ist der Haltekörper so ausgeführt, dass die Axialbohrung eine Langschaft-Aufnahme für den Schaft bildet. Unter Langschaft wird hierbei verstanden, dass die Tiefe der Aufnahmebohrung mindestens das Vierfache der Weite des Schaftes beträgt. Damit wird eine auch bei hohen Belastungen, insbesondere bei sportlicher Betätigung des Patienten, auch langzeitstabile Verankerung des Schafts im Haltekörper erreicht.

Die Erfindung bezieht sich weiter auf einen Satz umfassend mehrere Aufsteckmodule der vorstehend beschriebenen Art, wobei sich die Aufsteckmodule darin unterscheiden, dass die Gelenkköpfe unterschiedlich weit versetzt sind. Damit wird dem Operateur die Möglichkeit gegeben, aus einer Mehrzahl von Möglichkeiten die für den jeweiligen Befund des Patienten passende Größe herauszugreifen.

Die Erfindung wird nachfolgend unter Bezugnahme auf ein Ausführungsbeispiel näher erläutert, welches in der beigefügten Zeichnung dargestellt ist. Es zeigen:
- Fig.1: eine Aufsicht auf ein Aufsteckmodul gemäß einem Ausführungsbeispiel;
- Fig. 2: eine Schnittansicht des in Fig. 1 dargestellten Ausführungsbeispiels mit einer Befestigungsschraube;
- Fig. 3: eine Vergleichsdarstellung bezüglich des Versatzmaß zwischen dem Aufsteckmodul und einer konventionellen Ausführung; und
- Fig. 4: ein Röntgenbild, welches die Lage des erfindungsgemäßen Aufsteckmoduls im Femur zeigt.

Ein in seiner Gesamtheit mit der Bezugsziffer 1 versehenes Aufsteckmodul umfasst einen rohrförmigen Haltekörper 2 sowie einen Gelenkkopf 3. Der Gelenkkopf 3 ist im unteren Drittel seitlich an dem Haltekörper 2 angeordnet und ragt nach schräg oben ab.

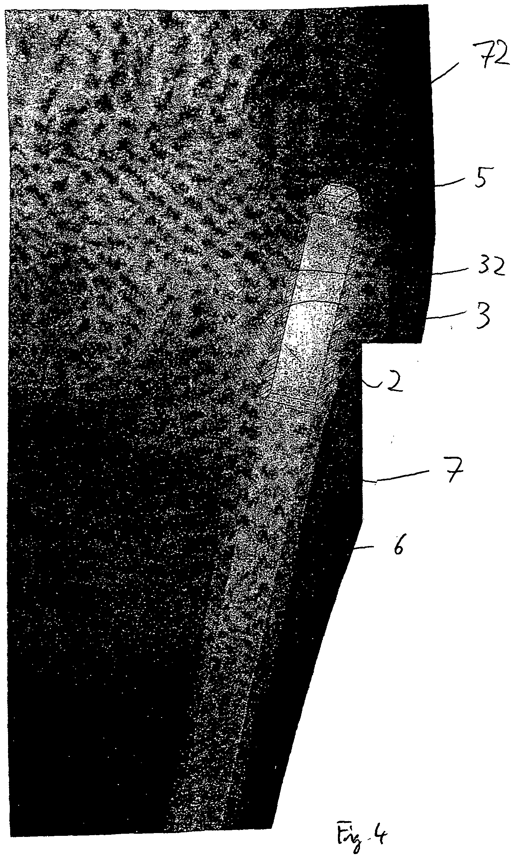

Der Haltekörper 2 weist an seinem unteren Ende eine Mündung für eine Aufnahmebohrung 20 auf. Die Aufnahmebohrung 20 ist so bemessen, dass ein freies Ende eines Durchsteckschafts 6 (siehe Fig. 4) spielfrei aufgenommen werden kann. Die Aufnahmebohrung 20 ist von zylindrischer Gestalt und ausgerichtet in Axialrichtung 26 des Haltekörpers 2. Die Aufnahmebohrung 20 weist eine Tiefe auf, die das Fünffache ihres Durchmessers beträgt. Das obere Ende der Aufnahmebohrung 20 ist begrenzt von einer Anlageschulter 22, die mit einer nach unten weisenden Vielfachradialverzahnung 23 versehen ist. Sie wirkt mit einer entsprechenden Gegenverzahnung an dem freien Ende des Durchsteckschaft 6 zusammen und sichert ihn so im aufgesteckten Zustand das Aufsteckmodul 1 gegenüber einer unerwünschten Verdrehung in Bezug auf den Durchsteckschaft 6.

Die Aufnahmebohrung 20 ist im oberen Bereich des Haltekörpers 2 entlang der Axialrichtung 26 fortgesetzt durch eine Stufenbohrung 21. Sie dient zur Aufnahme einer Sicherungsschraube 5, welche von oben in die Stufenbohrung 21 eingesetzt wird und mit ihrem Gewinde in den Durchsteckschaft 6 geschraubt wird und diesen somit sichert. Um die Sicherungsschraube 5 an einem unerwünschten Lösen zu hindern, ist seitlich ein aus Kunststoffmaterial gefertigtes Stoppelement 51 eingesetzt.

Der Gelenkkopf 3 ist einstückig mit dem Haltekörper 2 ausgebildet und weist in seinem äußeren, nach schräg oben abragenden Bereich eines Außenkonus 31 auf der. Diese fungiert als Aufnahme für einen Kugelkopf 32, die mit einem komplementären Innenkonus versehen ist und zur Implantation spielfrei aufgesetzt wird. Weiter ist an dem Gelenkkopf 3 ein nach schräg unten abragenden Vorsprung 4 ausgebildet. Er dient als zusätzliche Auflage des Haltestücks 2 im Bereich eines resezierten Schenkelhalses des Femurs 7, in welchem das Aufsteckmodul 1 mit dem Durchsteckschaft 6 implantiert ist.

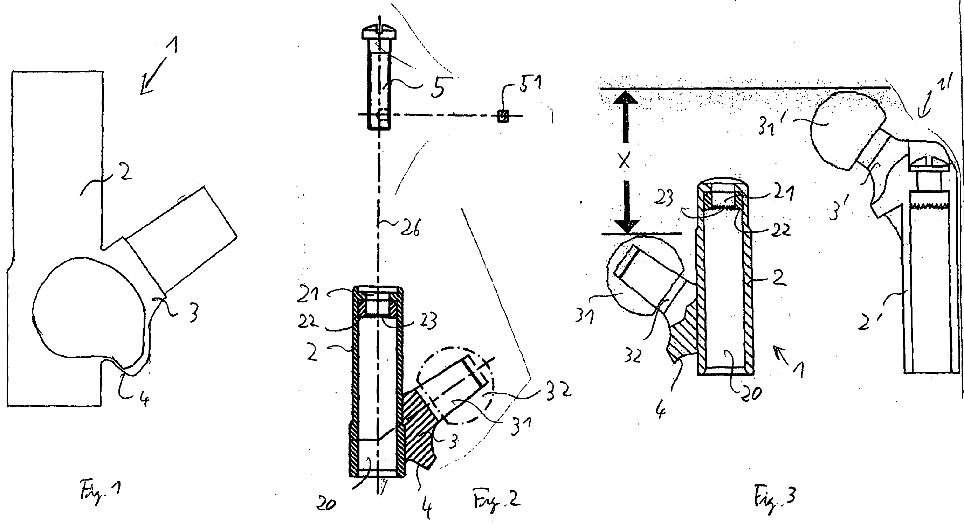

In Figur 3 ist der axiale Versatz des Gelenkkopfs 3 in Bezug auf ein herkömmliches Aufsteckmodul 1' dargestellt. Während bei dem herkömmlichen Aufsteckmodul 1' der Gelenkkopf 3' am oberen Ende des Haltekörpers 2' angeordnet ist und sich noch weiter oberhalb erstreckt, ist im Gegensatz dazu bei dem erfindungsgemäßen Aufsteckmodul 1 der Gelenkkopf 3 um ein Maß "X" nach unten versetzt. Der Gelenkkopf 3 befindet sich unterhalb des oberen Endes des Haltekörpers 2 in einer sozusagen versenkten Anordnung. Damit kann das erfindungsgemäße Aufsteckmodul 1 in all den Fällen für Abhilfe sorgen, in denen die Verwendung eines herkömmlichen Aufsteckmodul 1' nicht mehr möglich ist.

Ein solcher Fall ist in Figur 4 dargestellt. Sie zeigt die Röntgenaufnahme eines Hüftgelenks mit dem oberen Teil eines Femurs 7 einschließlich seines Schenkelhalses 72. In dem Femur 7 befindet sich bereits ein Durchsteckschaft 6 von einer vorhergehenden Implantation einer Kniegelenkendoprothese. Aufgrund pathologischer Veränderung ist der Kopf des Hüftgelenks nach unten gewandert und der Schenkelhals gebrochen. Es besteht daher der Bedarf an einer Hüftgelenkendoprothese. Aufgrund des gebrochenen Schenkelhalses 72 ist die Beinlänge verkürzt. Würde ein herkömmliches Aufsteckmodul 1' verwendet, so müsste der Durchsteckschaft 6 ausgetauscht werden. Mit dem erfindungsgemäßen Aufsteckmodul 1' kann die Beinverkürzung durch die versenkte Anordnung des Gelenkkopfs um den Versatz X ausgeglichen werden. Dies ermöglicht die Beibehaltung des vorhandenen Durchsteckschafts 6 und damit eine weitgehende Schonung des Knochens und des zugeordneten Bandapparats des Hüftgelenks.

## Patentansprüche

1. System aus einem zur Implantation in einem lang gestreckten Knochen (7) ausgebildeten Schaft (6) und einem Aufsteckmodul (1) zum Aufschieben auf ein freies Ende des Schafts (6), umfassend einen Haltekörper (2) mit einer Axialbohrung (20) für den Schaft (6) an dem unteren Ende des Haltekörpers (2) und einen Gelenkkopf (3) als ein Teil einer Gelenkendoprothese,
**dadurch gekennzeichnet, dass**
der Gelenkkopf (3) in Axialrichtung (26) in der Weise so weit von dem oberen Ende des Haltekörpers (2) nach unten versetzt ist, dass der Schaft (6) über den Gelenkkopf (3) nach oben hinausragt.

2. System nach Anspruch 1, **dadurch gekennzeichnet, dass** der Gelenkkopf (3) zusätzlich in eine Richtung quer zur axiale Richtung (26) versetzt ist.

3. System nach Anspruch 2, **dadurch gekennzeichnet, dass** der Gelenkkopf (3) nach anterior oder posterior aus der Ebene der Axialrichtung (26) gekippt ist.

4. System nach einem der Ansprüche 1 bis 3, **dadurch gekennzeichnet, dass** die Axialbohrung (20) zur Aufnahme des Schafts (6) als Sackloch ausgeführt ist.

5. System nach einem der Ansprüche 1 bis 3, **dadurch gekennzeichnet, dass** die Axialbohrung (20) als gestufte Durchgangsbohrung ausgeführt ist, wobei vorzugsweise eine Anlageschulter (22) vorgesehen ist und jenseitig davon die Weite der Bohrung (21) kleiner ist als die Weite des Schafts (6).

6. System nach Anspruch 4 oder 5, **dadurch gekennzeichnet, dass** am Grund des Sacklochs oder an der Anlageschulter (22) eine Radialverzahnung (23) und/oder ein Konus zur Verbindung angeordnet ist.

7. System nach Anspruch 6, **dadurch gekennzeichnet, dass** die Radialverzahnung (23) als eine Vielfachverzahnung ausgeführt ist, vorzugsweise mit einer Winkelabstufung von mindestens 1° und höchstens 5°.

8. System nach einem der vorangehenden Ansprüche,
**dadurch gekennzeichnet, dass** der Gelenkkopf (3) zweiteilig ausgeführt ist, und zwar vorzugsweise mit einem am Haltekörper (2) angeordneten Grundkonus (31) und einem aufsteckbaren Kopf (32).

9. System nach einem der vorangehenden Ansprüche,
**dadurch gekennzeichnet, dass** der Gelenkkopf (3) positionsvariabel versetzt an dem Haltekörper (2) angeordnet ist.

10. System nach einem der vorangehenden Ansprüche,
**dadurch gekennzeichnet, dass** die Axialbohrung (20) eine Langschaft-Aufnahme für den Schaft (6) bildet.

11. Satz umfassend ein System aus einem zur Implantation in einem lang gestreckten Knochen (7) ausgebildeten Schaft (6) und mehrere Aufsteckmodule zum Aufschieben auf ein freies Ende des Schafts (6), umfassend einen Haltekörper (2) mit einer Axialbohrung (20) für den Schaft (6) an dem unteren Ende des Haltekörpers (2) und einen Gelenkkopf (3) als ein Teil einer Gelenkendoprothese,
**dadurch gekennzeichnet, dass**
der Gelenkkopf (3) in Axialrichtung (26) in der Weise so weit von dem oberen Ende des Haltekörpers (2) nach unten versetzt ist, dass der Schaft (6) über den Gelenkkopf (3) nach oben hinausragt, wobei die Gelenkköpfe (3) unterschiedlich weit versetzt sind.

12. Satz nach Anspruch 11, **dadurch gekennzeichnet, dass** das Aufsteckmodul (1) nach einem der Ansprüche 2 bis 10 weitergebildet ist.

## Claims

1. System consisting of a shaft (6) designed for implantation in a long bone (7) and of a plug-on module (1) for pushing onto a free end of the shaft (6), comprising a holding body (2), with an axial bore (20) at the lower end of the holding body (2) for the shaft (6), and a joint head (3) as a part of a joint endoprosthesis, **characterized in that** the joint head (3) is offset downward in the axial direction (26) from the upper end of the holding body (2) sufficiently far and in such a way that the shaft (6) protrudes upward beyond the joint head (3).

2. System according to Claim 1, **characterized in that** the joint head (3) is additionally offset in a direction transverse to the axial direction (26).

3. System according to Claim 2, **characterized in that** the joint head (3) is tilted anteriorly or posteriorly from the plane of the axial direction (26).

4. System according to one of Claims 1 to 3, **characterized in that** the axial bore (20) for receiving the shaft (6) is designed as a blind hole.

5. System according to one of Claims 1 to 3, **characterized in that** the axial bore (20) is designed as a stepped through-bore, wherein an abutment shoulder (22) is preferably provided, beyond which the width of the bore (21) is smaller than the width of the shaft (6).

6. System according to Claim 4 or 5, **characterized in that** a radial toothing (23) and/or a cone for the connection are arranged at the bottom of the blind hole or on the abutment shoulder (22).

7. System according to Claim 6, **characterized in that** the radial toothing (23) is designed as a multiple toothing, preferably with an angle gradation of at least 1° and at most 5°.

8. System according to one of the preceding claims, **characterized in that** the joint head (3) is designed in two parts, i.e. preferably with a base cone (31) arranged on the holding body (2) and with a plug-on head (32).

9. System according to one of the preceding claims, **characterized in that** the joint head (3) is arranged on the holding body (2) so as to be variable in its offset position.

10. System according to one of the preceding claims, **characterized in that** the axial bore (20) forms a long shaft receiver for the shaft (6).

11. Kit comprising a system consisting of a shaft (6) designed for implantation in a long bone (7) and several plug-on modules for pushing onto a free end of the shaft (6), comprising a holding body (2), with an axial bore (20) at the lower end of the holding body (2) for the shaft (6), and a joint head (3) as a part of a joint endoprosthesis, **characterized in that** the joint head (3) is offset downward in the axial direction (26) from the upper end of the holding body (2) sufficiently far and in such a way that the shaft (6) protrudes upward beyond the joint head (3), wherein the joint heads (3) are offset to different extents.

12. Kit according to Claim 11, **characterized in that** the plug-on module (1) is further configured according to one of claims 2 through 10.

## Revendications

1. Système constitué d'une tige (6) réalisée pour l'implantation dans un os long (7) et d'un module d'enfichage (1) destiné à être enfoncé sur une extrémité libre de la tige (6), comprenant un corps de retenue (2) avec un alésage axial (20) pour la tige (6) au niveau de l'extrémité inférieure du corps de retenue (2) et une tête d'articulation (3) en tant que partie d'une endoprothèse d'articulation,
**caractérisé en ce que** la tête d'articulation (3) est décalée vers le bas par rapport à l'extrémité supérieure du corps de retenue (2) dans la direction axiale (26) dans une mesure telle que la tige (6) fasse saillie vers le haut au-dessus de la tête d'articulation (3).

2. Système selon la revendication 1, **caractérisé en ce que** la tête d'articulation (3) est en outre décalée dans une direction transversalement par rapport à la direction axiale (26).

3. Système selon la revendication 2, **caractérisé en ce que** la tête d'articulation (3) est basculée dans la direction antérieure ou postérieure à partir du plan de la direction axiale (26).

4. Système selon l'une quelconque des revendications 1 à 3, **caractérisé en ce que** l'alésage axial (20) pour recevoir la tige (6) est réalisé sous forme de trou borgne.

5. Système selon l'une quelconque des revendications 1 à 3, **caractérisé en ce que** l'alésage axial (20) est réalisé sous forme d'alésage traversant étagé, un épaulement d'appui (22) étant de préférence prévu et au-delà de celui-ci, la largeur de l'alésage (21) étant inférieure à la largeur de la tige (6).

6. Système selon la revendication 4 ou 5, **caractérisé en ce qu'**à la base du trou borgne ou au niveau de l'épaulement d'appui (22) est disposée une denture radiale (23) et/ou un cône en vue de la connexion.

7. Système selon la revendication 6, **caractérisé en ce que** la denture radiale (23) est réalisée sous forme de denture multiple, de préférence avec un étagement angulaire d'au moins 1° et d'au plus 5°.

8. Système selon l'une quelconque des revendications précédentes, **caractérisé en ce que** la tête d'articulation (3) est réalisée en deux parties, à savoir de préférence avec un cône de base (31) disposé au niveau du corps de retenue (2) et avec une tête enfichable (32).

9. Système selon l'une quelconque des revendications précédentes, **caractérisé en ce que** la tête d'articulation (3) est disposée de manière décalée avec une position variable au niveau du corps de retenue (2).

10. Système selon l'une quelconque des revendications précédentes, **caractérisé en ce que** l'alésage axial (20) forme un logement de tige allongée pour la tige (6).

11. Ensemble comprenant un système constitué d'une tige (6) réalisée pour l'implantation dans un os long (7) et de plusieurs modules d'enfichage destinés à être enfoncés sur une extrémité libre de la tige (6), comprenant un corps de retenue (2) avec un alésage axial (20) pour la tige (6) au niveau de l'extrémité inférieure du corps de retenue (2) et une tête d'articulation (3) en tant que partie d'une endoprothèse d'articulation,
**caractérisé en ce que**
la tête d'articulation (3) est décalée vers le bas par rapport à l'extrémité supérieure du corps de retenue (2) dans la direction axiale (26) dans une mesure telle que la tige (6) fasse saillie vers le haut au-dessus de la tête d'articulation (3), les têtes d'articulation (3) étant décalées à des distances différentes.

12. Ensemble selon la revendication 11, **caractérisé en ce que** le module d'enfichage (1) est perfectionné selon l'une quelconque des revendications 2 à 10.
